# EUROPEAN PATENT SPECIFICATION

(11) **EP 4 051 148 B1**
(45) Date of publication and mention of the grant of the patent: **03.09.2025**
(21) Application number: 20812164.0
(22) Date of filing: 28.10.2020
(51) Int. Cl.: A61B 18/12, A61B 18/14, A61B 18/00

(54) **ADJUSTMENT OF RENAL DENERVATION ENERGY DELIVERY**
EINSTELLUNG DER ENERGIEABGABE BEI EINER NIERENDENERVATION
AJUSTEMENT D'APPORT D'ÉNERGIE DE DÉNERVATION RÉNALE

(30) Priority: 31.10.2019 US 201962928860 P
(43) Date of publication of application: 07.09.2022
(73) Proprietor: Medtronic Ireland Manufacturing Unlimited Company, Dublin 2, D02 T380 (IE)
(72) Inventor: HETTRICK, Douglas, Santa Rosa, California 95403 (US); COATES, Paul, Santa Rosa, California 95403 (US)
(74) Representative: Zimmermann & Partner Patentanwälte mbB
(86) International application number: PCT/US2020/057770
(87) International publication number: WO 2021/086987

(56) References cited:
- WO-A2-2008/003058
- US-A1- 2012 101 538
- US-A1- 2017 105 783

## Description

### BACKGROUND

### 1. Field

This specification relates to a system, a device, a method and/or an apparatus for adjusting renal denervation energy delivery to prevent bradycardia in patients when performing renal denervation.

### 2. Description of the Related Art

The heart rate of adults at rest is approximately between 60 and 100 beats per minute. During renal denervation, a small proportion of patients suffer transient episodes of bradycardia arrhythmia. Bradycardia is when the heart beats at a slower than normal heart rate. If the patient has bradycardia, the heart beats fewer than 60 times a minute. Bradycardia can be a serious problem if the heart does not pump enough oxygen-rich blood to the body.

During renal denervation, a nurse, doctor, technician or other hospital staff (or "clinician") uses stimuli or energy, such as radiofrequency, ultrasound, cooling or other energy, to perform ablation within the renal arteries. This reduces activity of the nerves surrounding the vessel, which has been shown to result in decreased blood pressure, as well other potential benefits. The clinician may monitor the heart rate of the patient during renal denervation to prevent and/or detect bradycardia using separate standard medical equipment. In a subset of patients, the administration of renal denervation may cause bradycardia due to stimulation of the vagus nerve (or some other autonomic nerve distinct from the targeted sympathetic nerves), which travels close to the renal artery area, for example. If a slowing of the heart rate is detected, such that the slowing may result in bradycardia, the clinician may opt to stop the ablation procedure to prevent the continued reduction in the heart rate. If the slowing is significant, the clinician may administer a drug or pharmacology, such as atropine, to increase the heart rate and prevent further reduction. These actions to stop the procedure or administer therapy may prolong the procedure and/or cause the procedure to be aborted. These procedural adjustments, however, are not automatic and require the clinician to continue to monitor the heart rate of the patient, and in response, take steps, such as communicate with another clinician, to address the changes in the heart rate, which causes delay in effective treatment. The delay in treatment may lead to additional hemodynamic complications for the patient due to reduced cardiac output secondary to the low heart rate. In addition, reduced heart rate may cause reduced blood flow through the ablated vessels potentially leading to thermal damage to the vessel wall by the ablation system because these systems often rely on robust arterial blood flow to convectively cool the energy delivery elements, such as RF electrodes.

It may also be efficient to discontinue ablation to a particular electrode or electrodes following the detection of bradycardia because the bradycardia may be caused by only one or a few of several electrodes operating simultaneously. Accordingly, there is a need for a system, apparatus and/or method to automatically detect the heart rate of the patient and manage, adjust or otherwise control the energy output to prevent, detect and/or otherwise manage changes in the heart rate of the patient.

Document US 2012/0101538 A1 relates to devices, systems and methods for evaluation and feedback of neuromodulation treatment.

### SUMMARY

The invention is defined by the claims. In the following all methods of treatment are not claimed and are disclosed for illustrative purposes only.

In general, one aspect of the subject matter described in this specification is embodied in a therapeutic assembly for renal denervation. The therapeutic assembly includes a first sensor that is configured to continuously detect a first temperature or a first impedance at a first location of a wall of a vessel. The therapeutic assembly includes a first energy delivery element that is configured to delivery energy to a nerve in the wall of the vessel. The therapeutic assembly includes a processor coupled to the first sensor and the first energy delivery element. The processor is configured to determine a heart rate based on the first temperature or the first impedance at the first location of the wall of the vessel. The processor is configured to determine that the heart rate is less than a threshold heart rate that indicates a slowing of the heart rate or track a rate of change of the heart rate that indicates the slowing of the heart rate. The processor is configured to control the first energy delivery element to adjust the delivery of the energy to a nerve in the wall of the vessel.

These and other embodiments may optionally include one or more of the following features. The processor may be configured to turn off the first energy delivery element when the heart rate is less than the threshold heart rate. The therapeutic assembly may include a second energy delivery element. The second energy delivery element may be configured to deliver a second amount of energy to a second location on the wall of the vessel. The first energy delivery element may be configured to deliver a first amount of energy to a first location on the wall of the vessel.

The processor may be configured to power off the first energy delivery element and the second energy delivery element. The processor may be configured to cycle the first energy delivery element and the second energy delivery element to determine a cause of the slowing of the heart rate. The processor may be configured to power off one of the first energy delivery element or the second energy delivery element based on the cause of the slowing of the heart rate. The processor may be configured to power on the other one of the first energy delivery element or the second energy delivery element based on the cause of the slowing of the heart rate. The processor may be configured to provide on a display a notification or indication to a clinician that the heart rate is less than the threshold heart rate and obtain a confirmation to proceed to determine a cause of the slowing of the heart rate.

The therapeutic assembly may include a second sensor. The second sensor may be configured to detect a second temperature at a second location of the wall of the vessel. The processor may be configured to determine the heart rate further based on the second temperature at the second location of the wall of the vessel. The therapeutic assembly may include a catheter coupled to the first sensor and the first energy delivery element. The catheter is configured to be intravascularly inserted into the vessel. The vessel may be the renal artery and the first sensor may be coupled to the first energy delivery element. The therapeutic assembly may include a radio frequency generator. The radio frequency generator may be configured to deliver energy to the first energy delivery element. The energy may be a radiofrequency (RF) signal and the first energy delivery element may be an electrode.

In another aspect, the subject matter is embodied in a therapeutic assembly for renal denervation. The therapeutic assembly includes multiple sensors. The multiple sensors include a first sensor configured to detect a first temperature at a first location of a wall of a vessel. The therapeutic assembly includes multiple electrodes. The multiple electrodes are configured to delivery energy to the wall of the vessel. The therapeutic assembly includes a processor that is coupled to the multiple sensors and the multiple electrodes. The processor is configured to determine a heart rate based on the first temperature at the first location of the wall of the vessel. The processor is configured to determine that the heart rate is less than a threshold heart rate that indicates a slowing of the heart rate. The processor is configured to power off one of the plurality of electrodes to prevent bradycardia.

In another aspect, the subject matter is embodied in a method of managing a heart rate of a patient during renal denervation. The method includes measuring, by a sensor, sensor data at a location of a wall of a vessel. The method includes determining, by a processor, a heart rate of the patient based on the sensor data. The method includes determining, by the processor, that the heart rate is less than a threshold heart rate that indicates a slowing of the heart rate. The method includes controlling, by the processor and using one or more electrodes, an amount of energy delivered to the location of the wall of the vessel.

### BRIEF DESCRIPTION OF THE DRAWINGS

Other systems, methods, features, and advantages of the disclosure will be or will become apparent to one of ordinary skill in the art upon examination of the following figures and detailed description. It is intended that all such additional systems, methods, features, and advantages be included within this description. Component parts shown in the drawings are not necessarily to scale and may be exaggerated to better illustrate the important features of the present invention. In the drawings, like reference numerals designate like parts throughout the different views.
FIG. 1 shows an example conceptual illustration of the therapeutic assembly according to an aspect of the invention.
FIG. 2A shows an example renal denervation device of the therapeutic assembly of FIG. 1 in a low-profile delivery configuration according to an aspect of the invention.
FIG. 2B shows an example renal denervation device of the therapeutic assembly of FIG. 1 in an expanded deployed configuration according to an aspect of the invention.
FIG. 3 is a block diagram of an example generator of the therapeutic assembly of FIG. 1 according to an aspect of the invention.
FIG. 4 is a flow diagram of an example process for controlling the energy delivered to the one or more energy delivery elements of the therapeutic assembly of FIG. 1
FIG. 5 shows an example renal denervation device of the therapeutic assembly of FIG. 1 in the expanded deployed configuration within a blood vessel according to an aspect of the invention.
FIG. 6 is a flow diagram of an example process for determining the cause of the slowing of the heart rate using the therapeutic assembly of FIG. 1
FIG. 7 is a flow diagram of an example process for performing different actions at different threshold heart rates using the therapeutic assembly of FIG. 1
FIG. 8 shows a measured temperature at a treatment site using the therapeutic assembly of FIG. 1 according to an aspect of the invention.
FIG. 9 shows a measured impedance at a treatment site using the therapeutic assembly of FIG. 1 according to an aspect of the invention.
FIG. 10 shows the derivative of a measured temperature and a measured impedance using the therapeutic assembly of FIG. 1 according to an aspect of the invention.

### DETAILED DESCRIPTION

Disclosed herein are systems, devices, methods and/or apparatuses for a therapeutic assembly including a renal denervation device that controls an amount of energy delivered from each energy delivery element, such as an electrode, to prevent a slowing of the heart rate, such as bradycardia, during renal denervation. The renal denervation device has one or more integrated sensors that measure the temperature of the wall of the vessel, such as the renal artery, and/or the impedance and uses the temperature and/or impedance to determine the heart rate of the patient. If the renal denervation device detects that the heart rate is slowing such that bradycardia may occur, the renal denervation device may automatically adjust the amount of energy delivered through each of one or more energy delivery elements to prevent the slowing of the heart rate. The renal denervation device controls the amount of energy delivered, automatically, and as such, there is little or no delay in responding to the slowing of the heart rate to prevent bradycardia.

Other benefits and advantages include controlling each energy delivery element independently. The renal denervation device may have multiple energy delivery elements that are positioned at different locations within or along the wall of the vessel. The renal denervation device may control the delivery of energy to each of the energy delivery elements independently. The renal denervation device may turn on, turn off and/or adjust an amount delivered through each of the energy delivery elements, which allows the renal denervation device to optimize treatment of the blood pressure, while maintaining the heart rate within a normal heart rate range to prevent bradycardia.

Additionally, the renal denervation device may have one or more sensors. The one or more sensors may be integrated within the renal denervation device. The renal denervation device determines the patient's heart rate based on the sensor data, such as the temperature or impedance at the treatment site. And as such, the clinician does not need a separate device to monitor the heart rate of the patient. Since the clinician does not need to monitor a separate device, and then communicate to adjust the delivery of the energy, the adjustment of the energy delivered through the energy delivery element is automatic, which reduces delay in reacting to a slowing of the heart rate. By reducing the delay, the renal denervation device may adjust the delivery of the energy, promptly, and prevent the need to administer a drug, such as atropine, to increase the heart rate and to prevent bradycardia.

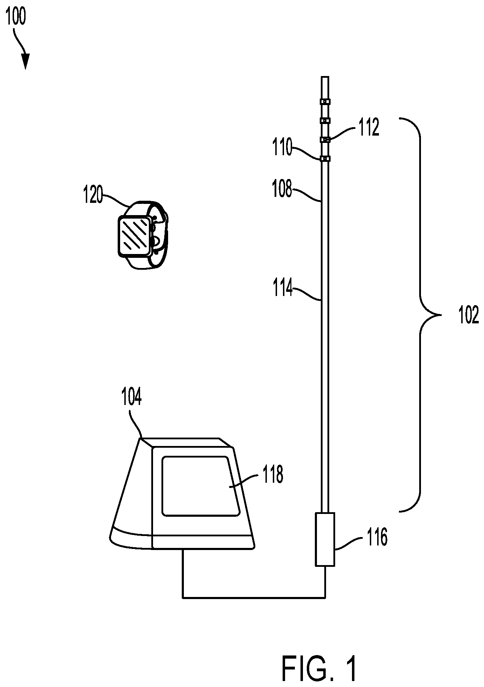

FIG. 1 shows the therapeutic assembly 100. The therapeutic assembly 100 performs renal denervation within the renal artery of a human patient. Renal denervation is a minimally invasive procedure to treat resistant hypertension. The therapeutic assembly 100 includes a renal denervation device 102 and/or a generator 104. The renal denervation device 102 may include any device that delivers energy or stimulus to a target nerve within a wall of a blood vessel, such as the renal nerve of the renal artery. The energy or stimulus may include, for example, at least one of a radio frequency stimulus, a thermal stimulus, a cryogenic stimulus, a microwave stimulus, an ultrasonic stimulus or other form of energy or stimulus.

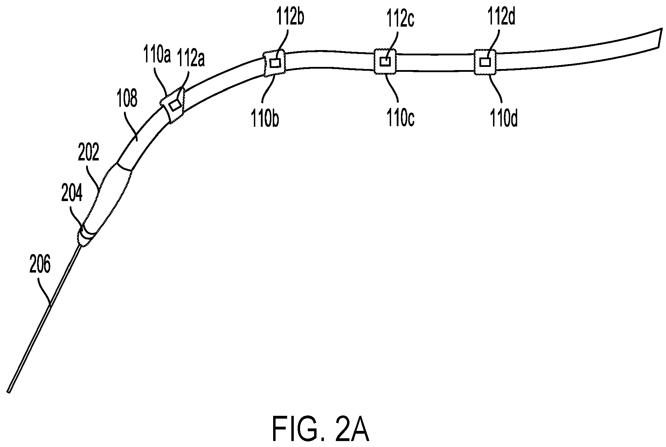

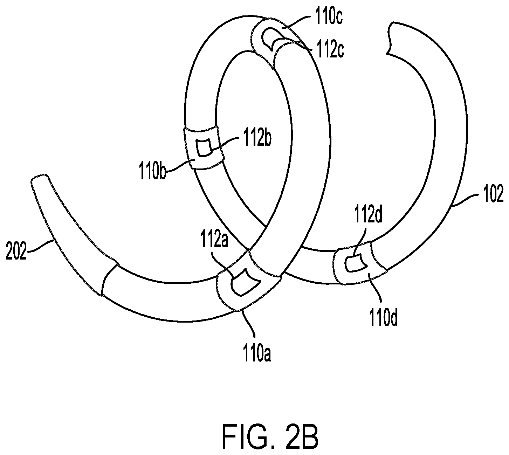

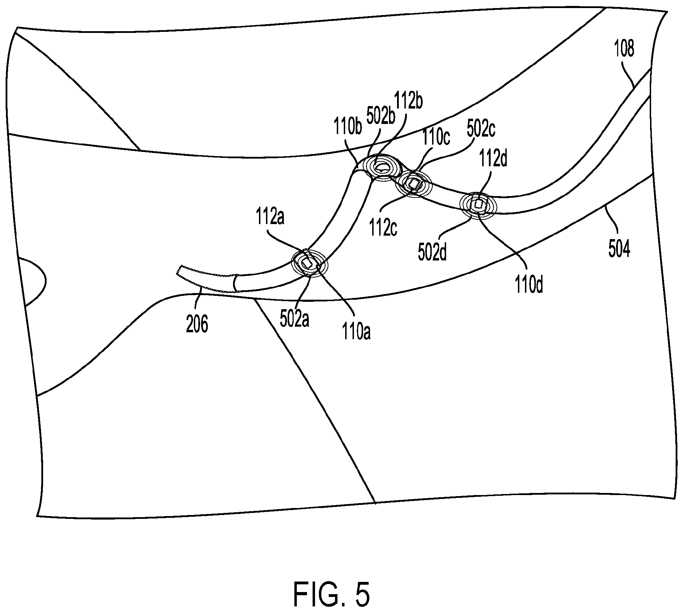

The renal denervation device 102 has a catheter 108, one or more energy delivery elements 110, such as an electrode, and/or one or more sensors 112. The renal denervation device 102 may have an elongated shaft 114 with a handle 116. The elongated shaft 114 with the handle 116 may be used to guide and/or advance a distal portion of the catheter 108 through the blood vessels of the patient, such as a human patient, to a target location of a blood vessel and remotely manipulate the distal portion of the catheter 108. The catheter 108 may be intravascularly delivered into the patient, e.g., a blood vessel of the patient, in a low-profile configuration, such as the substantially straight configuration shown in FIG. 1. The catheter 108 may be over a meter in length. Upon delivery to a target location within and along the blood vessel, the catheter 108 may be deployed into an expanded deployed configuration, such as a generally helical or spiral configuration or other suitable configuration, which the one or more energy delivery elements 110, such as one or more electrodes, may contact the blood vessel, as shown in FIG. 5 for example. In the expanded deployed state, the renal denervation device 102 may deliver energy at a treatment site and provide therapeutically-effective electrically and/or thermally induced denervation to a nerve within the wall of the blood vessel. FIGS. 2A-2B show the deployment of the renal denervation device 102. In particular, FIG. 2A shows the catheter 108 in the low-profile configuration, and FIG. 2B shows the catheter 108 in the expanded deployed configuration.

The catheter 108 may have a distal tip 202. The distal tip 202 points into the lumen of the blood vessel. The distal tip 202 may have a high density marker band 204. The high density marker band 204 allows a clinician to identify the distal tip 202 of the catheter 108 under fluoroscopy. The distal portion of the catheter 108 may be approximately 4 cm - 5 cm in length, and the distal tip 202 may be approximately 1 cm - 2 cm in length.

The catheter 108 may have a wire 206 within the lumen of the catheter 108. The distal tip 202 allows the wire 206 to extend out and away from the distal tip 202 when the catheter 108 is in the low-profile configuration and to be advanced through the blood vessels to the target location of the blood vessel. When the wire 206 is retracted within the distal tip 202 and into the catheter 108, the catheter 108 changes shape from the low-profile configuration, such as the substantially straight configuration, as shown in FIG. 2A for example, to the expanded deployed configuration, such as a generally helical or spiral configuration, as shown in FIG. 2B for example.

The renal denervation device 102 has one or more energy delivery elements 110. The one or more energy delivery elements 110 may include an electrode, such as a radiofrequency (RF) electrode, a radiofrequency (RF) probe, a thermal probe, a cryogenic probe, a microwave probe, an ultrasonic probe, an optical source or a chemical injector. The one or more energy delivery elements 110 may be positioned on the distal portion of the catheter 108. The one or more energy delivery elements 110 may include multiple energy delivery elements 110, such as the energy delivery elements 110a-d, as shown in FIGS. 2A, 2B and 5 for example, or any other N number of energy delivery elements 110. The energy delivery elements 110a-d may be arranged approximately 90 degrees apart relative to a longitudinal axis that runs through the center of the catheter 108 when in the spiral configuration. The energy delivery elements 110 may be spaced any suitable distance from each other, and the spacing may vary based on the application of the therapeutic assembly 100 and its intended use.

When there are multiple energy delivery elements 110, each energy delivery element 110 may deliver power independently, either simultaneously, selectively, and/or sequentially, to a treatment site. The multiple energy delivery elements 110 may deliver power among any desired combination of the one or more energy delivery elements 110.

The one or more energy delivery elements 110 may be introduced into and advanced along a blood vessel, such as the renal artery and may be positioned to contact the blood vessel in the expanded deployed configuration at different intervals and/or locations along the wall of the blood vessel. For example, a first energy delivery element 110a may contact the wall of the blood vessel 504 at a first location 502a, a second energy delivery element 110b may contact the wall of the blood vessel 504 at a second location 502b, a third energy delivery element 110c may contact the wall of the blood vessel 504 at a third location 502c and a fourth energy delivery element 110d may contact the wall of the blood vessel 504 at a fourth location 502d. The renal denervation device 102 may deliver energy through the one or more energy delivery elements 110 at the treatment sites and provide therapeutically-effective electrically- and/or thermally- induced denervation.

The renal denervation device 102 may include one or more sensors 112. The one or more sensors 112 may measure one or more parameters at or near the treatment site. The one or more parameters may include a temperature of one or more energy delivery elements 110 and/or a temperature at or near the treatment site. For example, the one or more sensors 112 may be a temperature sensor that measures the temperature at a location of the wall of the blood vessel. Each of the one or more sensors 112 may be coupled or integrated with a corresponding one of the one or more energy delivery elements 110. For example, the sensor 112a may be integrated with energy delivery element 110a, the sensor 112b may be integrated with energy delivery element 110b, the sensor 112c may be integrated with energy delivery element 110c and the sensor 112d may be integrated with energy delivery element 110d, as shown in FIG. 2B for example. The temperature may be used to interpolate the heart rate of the patient.

In another example, the one or more sensors 112 may be another type of sensor that measures a different parameter, such as an impedance, pressure, optical, flow, or amount of chemical. The impedance may be measured from one electrode to another electrode. The therapeutic assembly 100 may similarly use the different parameter, such as the impedance, pressure, optical, flow or amount of chemical to determine the heart rate. Other sensors or methods may also be developed to monitor the heart rate directly via the renal denervation device 102.

The one or more sensors 112 may be proximate to or within the energy delivery element 110. For example, the energy delivery element 110 may be an electrode, which has two wires. One wire may be made from copper and the other may be made from a copper-nickel allow. The wires may both transmit the signal from the sensor 112 and also convey the energy to the energy delivery element.

The signal may be a temperature signal that indicates the temperature of the blood vessel. The two wires may measure the temperature through a thermocouple effect. The two wires may have a voltage gap, and as the temperature changes due to the change in blood flow at the treatment site, the amount of voltage across the voltage gap changes. For example, when there is more blood flowing at the treatment site, there is a cooling effect, which results in a decrease in temperature, and when there is less blood flowing at the treatment site, there is a warming effect, which results in an increase in temperature. The amount of voltage across the voltage gap may be measured and may be associated to a temperature at the treatment site.

In some implementations, the one or more sensors 112 may measure the temperature at an independent location away from the one or more energy delivery elements 110. For example, the therapeutic assembly 100 may have an independent heating element or other additional device in place solely to be used to measure the temperature variation associated with the changes in the blood flow. This heating element may be independent of the one or more energy delivery elements 110 that heat the tissue, and as such, the renal denervation device 102 may determine the heart rate even when the RF energy is not being delivered to the tissue, e.g., a continuous heart rate measurement. This additional device may have an appendage that branches off or may have the sensor be attached to the outside of the catheter 108 and be connected back and integrated with the power source.

In some implementations, the one or more sensors 112 may measure or calculate an impedance at or near the treatment site. For example, the one or more sensors 112 may measure the impedance from a first energy delivery element to a second energy delivery element, such as from an ablation electrode to a dispersive electrode. In another example, the one or more sensors 112 may measure the voltage across the voltage gap of the two wires and calculate the impedance from the current and measured voltage. The renal denervation device 102 may use the temperature, the impedance and/or other parameter detected or obtained from the sensor to monitor the heart rate.

The therapeutic assembly may include a heart rate monitoring device 120. The heart rate monitoring device 120 may be a personal medical device, a wearable device, such as a smartwatch, or other device that measures the heart rate of a patient. The heart rate monitoring device 120 may sense or detect the heart beat and/or heart rate of the patient and communicate the heart beat and/or the heart rate of the patient to the renal denervation device 102 and/or the generator 104 via a wired or wireless network, such as a local area network (LAN), a wide area network (WAN), a cellular network, a digital short-range communication (DSRC), the Internet, or a combination thereof.

The therapeutic assembly includes a generator 104. The generator 104 may be a radio frequency generator or other generator that delivers a denervation stimulus or energy through the one or more energy delivery elements 110 to the wall of the blood vessel at the treatment location. The denervation stimulus may include a non-electric stimulus, for example, a chemical agent, optical stimulus, a thermal stimulus, a cooling stimulus, a microwave stimulus or other form of stimuli. The generator 104 may have a cable, an electrical lead and/or wire that is electrically conductive and runs through the catheter 108 within a lumen and is electrically coupled with the one or more energy delivery elements 110. In some implementations, the generator 104 may have separate leads and/or wires that electrically couple with a corresponding energy delivery element 110 of the one or more energy delivery elements 110 so that each energy delivery element 110 may operate independently of the others. For example, the generator 104 may have multiple separate channels, such as four radio frequency (RF) channels to deliver RF energy independently to the one or more energy delivery elements 110a-d and control and monitor each energy delivery element 110a-d independently. The generator 104 may generate energy that ultimately is transmitted through the electrical lead to the one or more energy delivery elements 110.

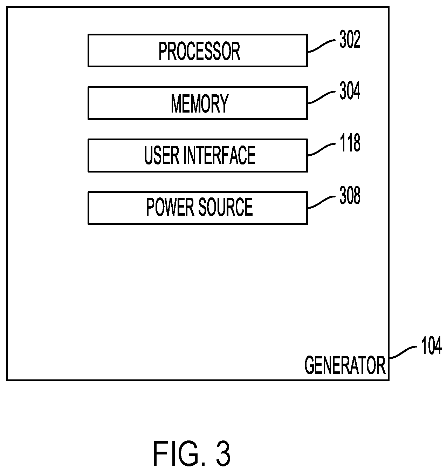

The generator 104 may have one or more processors 302, a memory 304, a user interface 118 and/or a power source 308, as shown in FIG. 3 for example. The one or more processors 302 may be electrically coupled to the memory 304, the user interface 118 and/or the power source 308. The one or more processors 302 may include one or more controllers that obtain a temperature signal and/or an impedance signal that indicates the temperature and/or impedance at the treatment site, respectively, and determines a heart rate based on the temperature signal and/or impedance signal. Once the heart rate is determined, the one or more processors 302 may control a state of each of the one or more energy delivery elements 110 and the amount of energy delivered to each of the one or more energy delivery elements 110 by the power source 308. The one or more processors may be coupled to the memory 304 and execute instructions that are stored in the memory 304.

The generator 104 may have a memory 304. The memory 304 may be coupled to the one or more processors 302 and store instructions that the one or more processors 302 executes. The memory 304 may include one or more of a Random Access Memory (RAM), Read Only Memory (ROM) or other volatile or non-volatile memory. The memory 304 may be a non-transitory memory or a data storage device, such as a hard disk drive, a solid-state disk drive, a hybrid disk drive, or other appropriate data storage, and may further store machine-readable instructions, which may be loaded and executed by the one or more processors 302.

The generator 104 may have a power source 308, such as a RF generator or other electrical source. The power source 308 provides a selected form and magnitude of energy for delivery to the treatment site via the renal denervation device 102. The generator 104 may have a user interface 118. The generator 104 may receive input, such as the selected form and the magnitude of energy to be delivered to each of the one or more energy delivery elements 110, via the user interface 118.

The user interface 118 may include an input/output device that receives user input from a user interface element, a button, a dial, a microphone, a keyboard, or a touch screen. The user interface 118 may provide an output to an output device, such as a display, a speaker, an audio and/or visual indicator, or a refreshable braille display. The output device may display an alert or notification or other information to the clinician and/or to confirm status and/or commands from the clinician. The output device may be an audio output device that outputs an audio indicator that indicates the notification or information to be provided to the clinician.

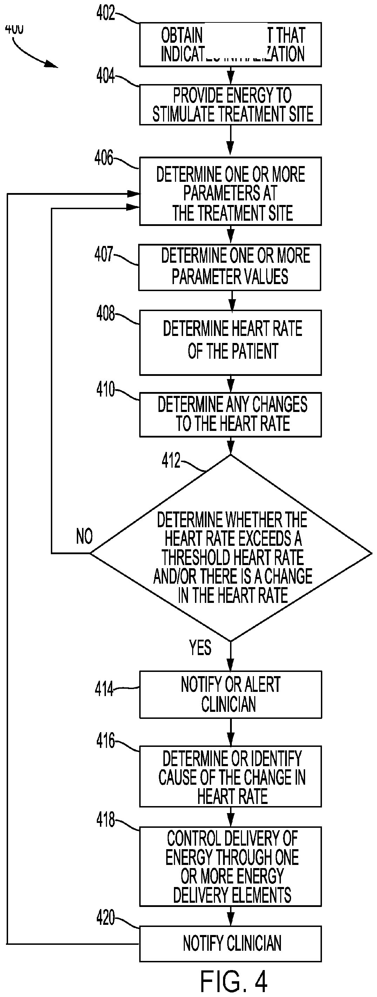

FIG. 4 is a flow diagram of a process 400 for controlling the energy delivered to the one or more energy delivery elements 110 to prevent bradycardia. One or more computers or one or more data processing apparatuses, for example, the processor 302 of generator 104 of the therapeutic assembly 100 of FIG. 1, appropriately programmed and/or using one or more other components, such as the one or more sensors 112 and/or the one or more energy delivery elements 110, may implement the process 400.

The therapeutic assembly 100 may include a generator 104, which controls the delivery of energy to the one or more energy delivery elements 110 of the renal denervation device 102. The generator 104 of the therapeutic assembly 100 receives user input that indicates initialization of the renal denervation device 102 (402). The generator 104 may receive the user input, which may be an indication to power-on the generator 104, via the user interface 118, which causes the generator 104 to power-on or initialize to deliver energy to the renal denervation device 102 and through the one or more energy delivery elements 110 to one or more treatment sites within the blood vessel 504.

Once powered-on, the therapeutic assembly 100 may deliver a first amount of energy through the one or more energy delivery elements 110 (404). The generator 104 may linearly or non-linearly ramp up or increase the amount of energy to the first amount of energy during the start-up phase until the amount of energy reaches the first amount of energy. The generator 104 may deliver the first amount of energy to each of the multiple energy delivery elements 110a-b when there are multiple energy delivery elements 110a-b, such as the first energy delivery element 110a, the second energy delivery element 110b, the third energy delivery element 110c and/or the fourth energy delivery element 110d, as shown in FIG. 5 for example. The multiple energy delivery elements 110a-d may be arranged to contact the wall of the blood vessel and at approximately 90 degree angles relative to a longitudinal axis that runs through the center of the spiral or helical configuration when the catheter 108 is in the expanded deployed state within the blood vessel.

During delivery of the energy, the therapeutic assembly 100 may measure, detect, obtain or determine one or more parameters at the treatment site (406). The one or more parameters may be the temperature of the one or more energy delivery elements 110, the temperature at or near the treatment site and/or the impedance at or near the treatment site. The therapeutic assembly 100 may measure, detect, obtain or determine a single parameter or multiple parameters at the treatment site.

The therapeutic assembly 100 may use one or more sensors 112 to measure the temperature of the one more energy delivery elements 110, the temperature at the treatment site along the wall of the blood vessel and/or measure the impedance at the treatment site. For example, the therapeutic assembly 100 may measure the voltage change across a voltage gap between two wires within an energy delivery element to determine the temperature. In another example, the therapeutic assembly 100 may measure the impedance of a signal transmitted between two energy delivery elements or sensors, such as the impedance of the signal between an energy delivery element and a sensor, to determine the impedance or use the voltage across the voltage gap and current to calculate the impedance.

The one or more sensors 112 may include multiple temperature sensors or multiple impedance sensors each positioned within a corresponding energy delivery element 110. Each of the multiple temperature sensors or the multiple impedance sensors may independently measure the temperature or the impedance, respectively, at the corresponding location along the wall of the blood vessel 504 that is in contact with the temperature sensor or the impedance sensor, respectively. For example, the energy delivery element 110a may be coupled with a sensor 112a at a first location 502a along the blood vessel 504 and the energy delivery element 110b may be coupled with another sensor 112b at a second location 502b along the blood vessel 504. The sensor 112a may measure a first temperature or the first impedance at the first location and the other sensor 112b may measure a second temperature or the second impedance at the second location 502b. Any number of sensors 112 may be used to compute the temperature or the impedance at any number of locations.

The therapeutic assembly may calculate or determine one or more parameter values related to the one or more parameters (407). The one or more parameter values are related to, correspond to and/or are based on the one or more parameters. The one or more parameter values may be calculated from the one or more parameters. For example, the one or more parameter values may be a peak (or maximum inflection point), a valley (or minimum inflection point), a cycle time (or frequency or period) and/or a derivative (or rate of change) of the corresponding parameter, such as the temperature or impedance at the treatment site.

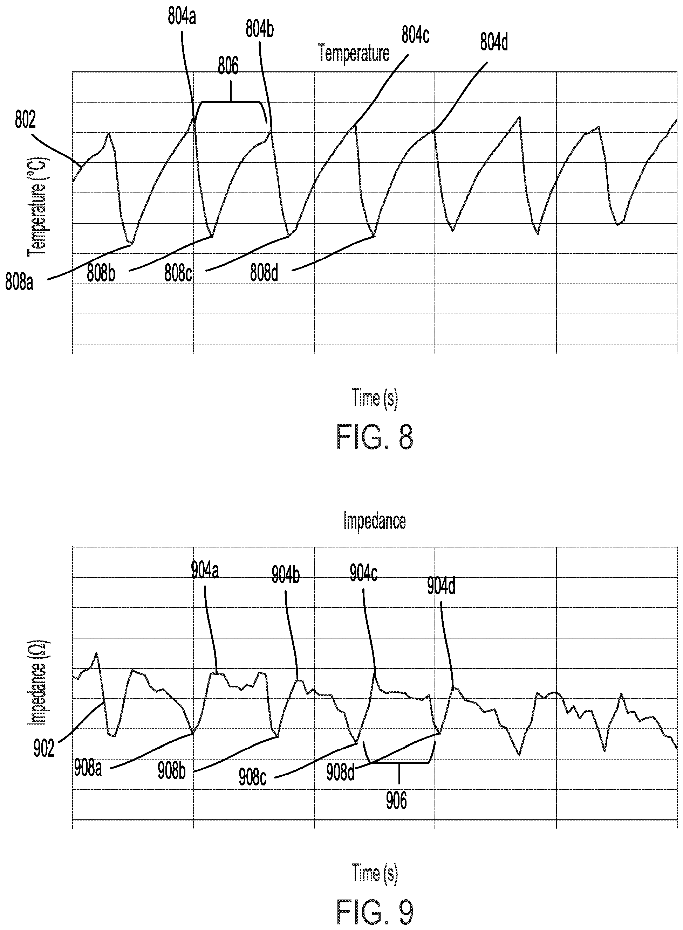

The therapeutic assembly 100 may obtain, detect or determine temperature signals that indicate the temperature at the treatment site using the one or more sensors 112 of the renal denervation device 102, as shown in FIG. 8 for example, and/or may obtain, detect or determine the impedance signals that indicate the impedance at the treatment site using the one or more sensors 112 of the renal denervation device 102, as shown in FIG. 9 for example. The therapeutic assembly 100 may use the temperature signal and/or the impedance signal to calculate other parameter values that correspond with the temperature signal and/or the impedance signal.

The temperature may be determined at multiple locations along the wall of the blood vessel by multiple sensors or may be determined at a single location along the wall of the blood vessel by a single sensor. The therapeutic assembly 100 may calculate or determine when the temperature 802 reaches one or more peak temperatures 804a-d based on the temperature 802 over a period of time. The one or more peak temperatures 804a-d may be a maximum inflection point where the temperature 802 reaches a maximum and changes from an increasing temperature to a decreasing temperature over the period of time. The therapeutic assembly 100 may calculate or determine when the temperature 802 reaches one or more valley temperatures 808a-d. The one or more valley temperatures 808 may be a minimum inflection point where the temperature 802 reaches a minimum and changes from a decreasing temperature to an increasing temperature over the period of time.

Once the therapeutic assembly 100 calculates or determines when the temperature 802 reaches two or more peak temperatures 804a-d and/or two or more valley temperatures 808a-d, the therapeutic assembly 100 may measure the time period between two or more sequential peak temperatures 804a-d or two or more sequential valley temperatures 808a-d. The calculated or determined time period may be associated with and correspond to the temperature change cycle 806. A sequential peak temperature is a subsequent peak temperature that immediately follows a current peak temperature, and a sequential valley temperature is a subsequent valley temperature that immediately follows a current valley temperature.

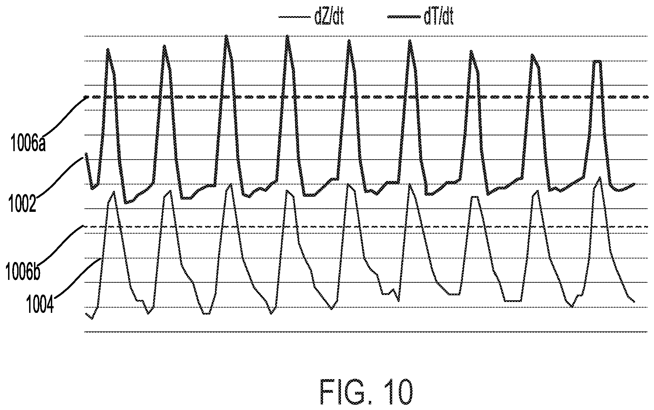

The therapeutic assembly 100 may calculate a first or a second derivative of the temperature that indicates the rate of change of the temperature. For example, the therapeutic assembly 100 may calculate a first derivative 1002 of the temperature 802, as shown in FIG. 10 for example. The first derivative 1002 may be the slope between two or more temperatures measured at different times, which may indicate the rate of change of the temperature 802.

The impedance may be measured by a sensor 112 or other sensor, such as a patch sensor, that receives an impedance signal from an energy delivery element 110. The therapeutic assembly 100 may calculate or determine when the impedance 902 reaches one or more peak impedances 904a-d. The one or more peak impedances 904a-d may be a maximum inflection point where the impedance 902 increases to a maximum and subsequently decreases. The therapeutic assembly 100 may calculate or determine when the impedance 902 reaches one or more valley impedances 908a-d. The one or more valley impedances 908a-d may be a minimum inflection point where the impedance 902 decreases to a minimum and subsequently increases.

Once the therapeutic assembly 100 calculates or determines when the impedance 902 reaches two or more peak impedances 904a-d and/or two or more valley impedances 908a-d, the therapeutic assembly 100 may measure the time period between two or more sequential peak impedances 904a-d and/or two or more sequential valley impedances 908a-d. The calculated or determined time period may be associated with and correspond to the impedance change cycle 906. A sequential peak impedance is a subsequent peak impedance that immediately follows a current peak impedance, and a sequential valley impedance is a subsequent valley impedance that immediately follows a current valley impedance. The peaks and valleys of the temperature and/or impedance reflect the contraction and relaxation of the heart muscle, such as between systole and diastole. By measuring the cycle time of the temperature and/or impedance, the therapeutic assembly 100 may calculate the heart rate.

The therapeutic assembly 100 may calculate a first or a second derivative of the impedance that indicates the rate of change of the impedance. For example, the therapeutic assembly 100 may calculate a first derivative 1004 of the impedance 902, as shown in FIG. 10 for example. The first derivative 1004 may be the slope between two or more impedances measured at different times, which may indicate the rate of change of the impedance 902.

The therapeutic assembly determines the heart rate of the patient (408). The heart rate of the patient may be determined based on the one or more parameters and/or the one or more parameter values, such as based on the temperature, the impedance and/or one or more parameter values calculated or determined from the temperature and/or the impedance. The heart rate of the patient may be determined from a single parameter, such as the temperature or impedance, a single parameter value, such as the temperature change cycle or impedance change cycle, or a combination of one or more parameters and/or one or more parameter values, such as the temperature change cycle and the impedance change cycle and/or the first derivatives of the temperature and the impedance.

The blood flow within the renal artery is highly pulsatile, and thus, the temperature and/or impedance within the renal artery oscillates and/or rhythmically varies. And so, when the heart is pumping, during systole, which is the phase of the heartbeat when the heart muscle contracts and pumps blood from the chambers into the arteries, there is a pulse of blood through the blood vessel that enhances the heat transfer and cools the one or more energy delivery elements 110, the treatment site and/or the one or more sensors 112, which defines a minimum temperature of the cycle. Moreover, as the pulse of blood flows through the blood vessel the vessel diameter dilates, which may cause the energy delivery element 110 to have less contact area with the wall of the vessel and more contact area with the blood, which has less impedance than the wall of the vessel, and thus, there is less impedance and defines a minimum impedance of the cycle. During diastole, which is the phase of the heartbeat when the heart muscle relaxes and allows the chambers to fill with blood, blood flow is at a minimum and cooling is at its least effective, which defines a maximum temperature of the cycle. Moreover, as the blood is withdrawn into the chambers of the heart muscle, the vessel diameter may constrict, which may cause the energy delivery element 110 to have more contact area with the wall of the vessel, which has more impedance than the blood, and less contact area with the blood, and thus, there is more impedance and defines a maximum impedance of the cycle. Thus, the temperature change cycle and/or impedance change cycle may indicate the heart rate because each temperature change cycle 806 or each impedance change cycle 906 may be reflective of a heartbeat and the number or amount of cycles of the temperature or the impedance per unit or period of time may be reflective of the heart rate.

In some implementations, the therapeutic assembly may use a first or second derivative or other statistical or mathematical measure of the temperature and/or impedance to determine the heart rate. The other statistical or mathematical measures may include when the temperature or impedance cycles past a threshold value, such as an average, or when a slope or tangent of the temperature values or impedance values invert, which may identify a minimum temperature inflection point (or valley) and/or a maximum temperature inflection point (or peak). The temperature signal and/or the impedance signal may be collected at a frequency of approximately 5 Hz or faster so that enough data is collected to determine the heart rate.

For example, after the therapeutic assembly 100 determines or calculates the first derivative 1002 of the temperature 802 and/or the first derivative 1004 of the impedance 902, as shown in FIG. 10, the therapeutic assembly 100 may obtain one or more corresponding thresholds 1006a-b and compare the first derivative 1002 of the temperature 802 and/or the first derivative 1004 of the impedance 902 to the one or more corresponding thresholds 1006a-b. The threshold 1006a may be a threshold temperature rate (e.g., degrees Celsius or Fahrenheit / second), and the threshold 1006b may be a threshold impedance rate (e.g., ohms / second). The therapeutic assembly 100 may determine when the first derivative 1002 of the temperature 802 increases past the threshold temperature rate and/or when the first derivative 1004 of the impedance 902 increases past the threshold impedance rate to identify when a heartbeat occurs. The therapeutic assembly 100 may divide the number of heart beats by the duration of the detection interval to calculate the heart rate. In another example, the therapeutic assembly 100 may similarly use the second derivative of the temperature 802 and/or the second derivative of the impedance 902 to compare with one or more corresponding thresholds to detect a heartbeat, and subsequently, use the heartbeat to calculate a heart rate. In some implementations, the therapeutic assembly 100 may use low pass filtering of the signal with a cutoff of, for example, approximately 20 or 30 Hz to increase the performance of the detection of the signal.

In some implementations, the therapeutic assembly 100 may obtain the heart rate of the patient from the heart rate monitoring device 120. For example, the heart rate monitoring device 120 may provide heartbeat signal to the generator 104, and the generator 104 may calculate the heart rate based on the number of heart beats over the duration of the detection interval. In another example, the heart rate monitoring device 120 may determine the heart rate and the generator 104 may receive a heart rate signal that indicates the heart rate of the patient from the heart rate monitoring device 120.

The therapeutic assembly 100 may determine any changes in the heart rate of the patient (410). The therapeutic assembly 100 compares the heart rate over a period of time to identify a trend or direction of the heart rate over the period of time, such as a period of approximately 5 seconds. If the heart rate increases over the period of time, then the therapeutic assembly 100 may determine that the heart rate is increasing, and if the heart rate decreases over the period of time, the therapeutic assembly 100 may determine that the heart rate is decreasing. In some implementations, the therapeutic assembly calculates the changes in the heart rate. If the slope is positive, the heart rate is increasing, and if the slope is negative, the heart rate is decreasing. If the slope is flat or approximately 0, the heart rate may be constant.

The therapeutic assembly 100 determines whether the heart rate exceeds a threshold heart rate and/or there is a change in the heart rate (412). The therapeutic assembly may establish a normal heart rate range, which may be an average of the heart rate of the patient over a period of time when the patient is normal or not under the effects of renal denervation. The therapeutic assembly 100 may use the heart rate monitoring device 120 to obtain the heart rate even when energy is not delivered to the tissue.

The therapeutic assembly 100 may determine that the heart rate is less than the threshold heart rate and/or is slowing. The threshold heart rate may be pre-configured, user-configured, obtained from another device, and/or an approximation or estimation of a reduction of a percentage of the normal heart rate of the patient over a period of time, such as approximately 7%-12% of the normal heart rate of the patient. The normal heart rate may have been previously stored in the memory 304 and later obtained by the processor 302 from the memory 304.

The therapeutic assembly 100 compares the heart rate to the threshold heart rate. The therapeutic assembly 100 may identify that the heart rate is less than the threshold heart rate and/or is decreasing and may perform different actions, such as notify the clinician via the user interface 118, turn off or disable the delivery of the energy to the one or more energy delivery elements 110, and/or identify the one or more energy delivery elements 110 that are causing the slowing of the heart rate when the heart rate is less than the threshold heart rate. In some implementations, the therapeutic assembly 100 may only perform the one or more of the different actions when the heart rate is less than the threshold heart rate and not increasing because if the heart rate is increasing the current course of action or remedy may be effective and does not necessarily need to change. The therapeutic assembly 100 may perform the different actions when different heart rate thresholds are exceeded.

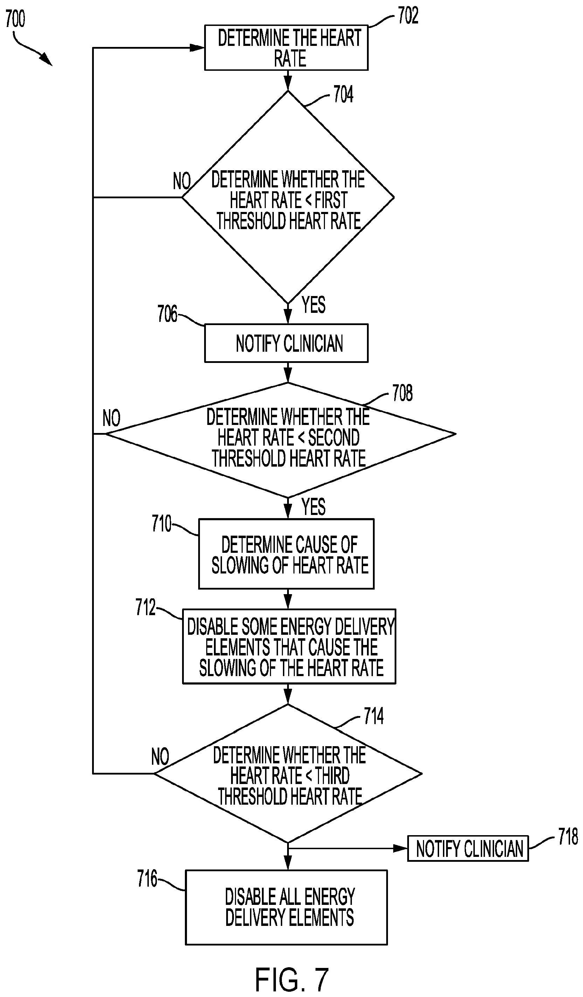

Otherwise, when the heart rate is not less than the threshold heart rate and/or the heart rate is increasing, the therapeutic assembly 100 may continue to monitor the one or more parameters at the treatment site (406). FIG. 7 further describes the implementation of the different actions when the different heart rate thresholds are exceeded.

In some implementations, when the therapeutic assembly 100 detects that the heart rate is greater than a threshold heart rate, which is greater than the normal heart rate, the therapeutic assembly 100 may decrease delivery of energy to the one or more energy delivery elements 110 to reduce the temperature of the one or more energy delivery elements 110. When the heart rate is greater than the threshold heart rate, this may indicate that the patient is in excessive pain, that the delivery of energy should be stopped and/or that the sedation level of the patient should be checked.

When the heart rate exceeds the threshold heart rate and/or there is a change in the heart rate, the therapeutic assembly 100 may notify the clinician (414). For example, when the heart rate is less than the threshold heart rate and/or is slowing, the notification may indicate that there is a slowing of the heart rate, that the heart rate is less than the threshold heart rate and/or that bradycardia may be possible or imminent. The notification may indicate a course of action for the clinician to implement or indicate to the clinician that the therapeutic assembly 100 may turn off or otherwise adjust or control the delivery of energy through the one or more energy delivery elements 110 to the treatment site. In some implementations, the therapeutic assembly 100 may request confirmation via user input from the clinician to allow the therapeutic assembly 100 to turn off or otherwise adjust or control the delivery of energy through the one or more energy delivery elements 110 to the treatment site prior to changing the settings of the delivery of the energy to the treatment site.

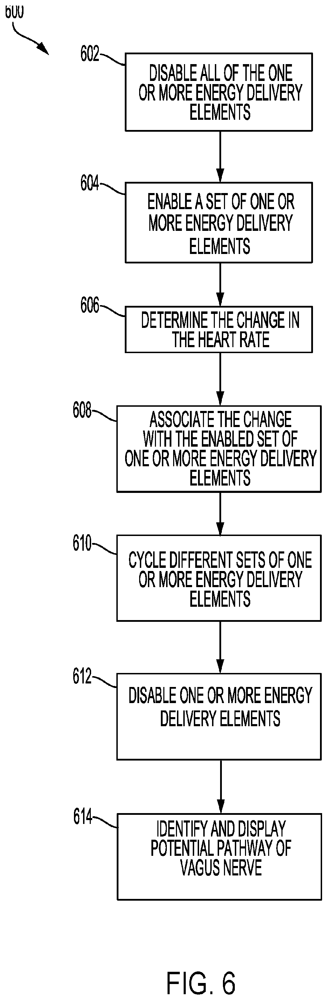

The therapeutic assembly 100 may determine or identify the one or more energy delivery elements 110 that are causing the heart rate to exceed the threshold heart rate (416). For example, the energy being delivered through the one or more energy delivery elements 110 may cause the heart rate to decrease below the threshold heart rate, and thus, the therapeutic assembly 100 may cycle through the one or more energy delivery elements 110 to identify the cause of the slowing of the heart rate. FIG. 6 further describes the process 600 of determining the cause of the slowing of the heart rate. By identifying the one or more energy delivery elements 110 that cause the heart rate to decrease, the therapeutic assembly 100 may turn off the particular energy delivery element 110 to prevent bradycardia while continuing treatment at the treatment site with the other of the one or more energy delivery elements 110.

The therapeutic assembly 100 controls the delivery of energy through the one or more energy delivery elements 110 (418). The therapeutic assembly 100 may control the delivery of energy in response to user input that confirms that the clinician would like to proceed with adjustment of the energy to the treatment site or may be done automatically. Since the one or more energy delivery elements 110 may be independently controlled, the therapeutic assembly 100 may turn off, disable or otherwise stop delivery of energy to all or some of the one or more energy delivery elements 110. For example, the therapeutic assembly 100 may only turn off, disable or otherwise stop delivery of energy to the set of energy delivery elements that are identified as causing the slowing of the heart rate. In another example, the therapeutic assembly 100 may turn off, disable or otherwise stop delivery of energy to all of the one or more energy delivery elements 110. In some implementations, the control of the delivery of energy may include adjusting the amount of energy that is delivered to each of the one or more energy delivery elements 110, such as decreasing or increasing the amount of energy that is delivered. For example, the therapeutic assembly 100 may decrease the amount of energy delivered by one or more energy delivery elements 110 from a first amount to a second amount that is less than the first amount to prevent bradycardia.

Once the therapeutic assembly 100 controls the delivery of the energy, such as to stop the delivery of the energy, the therapeutic assembly 100 may notify the clinician (420). The notification may indicate that the renal denervation has stopped or has been increased or decreased due to the slowing of the heart rate. This allows the clinician to wait for the heart rate to recover or apply atropine or other treatment or pharmacology to artificially increase the heart rate. The therapeutic assembly 100 may continue to monitor the parameters that correspond to the heart rate after the therapeutic assembly 100 has notified the clinician and/or has adjusted or controlled the energy delivery (406).

FIG. 6 is a flow diagram of a process 600 for determining the cause of the slowing of the heart rate. One or more computers or one or more data processing apparatuses, for example, the processor 302 of the therapeutic assembly 100 of FIG. 1, appropriately programmed, may implement the process 600. The therapeutic assembly 100 may determine the cause of the slowing heart rate in response to the heart rate slowing and/or may proactively identify any actions that may cause an eventual slowing of the heart rate.

When the therapeutic assembly 100 determines that the heart rate is less than the threshold heart rate, the heart rate is slowing and/or during initialization, the therapeutic assembly 100 may disable, turn off or leave off all of the one or more energy delivery elements 110 to ensure that no energy is being delivered to the treatment site and identify the cause or any potential actions that may cause a slowing of the heart rate (602). The generator 104 may stop or prevent the power source 308 from providing energy to the electrical leads that connect with the one or more energy delivery elements 110, which stops any treatment at the treatment site. For example, the therapeutic assembly 100 may power off a first energy delivery element 110a, a second energy delivery element 110b, a third energy delivery element 110c and a fourth energy delivery element 110d.

The therapeutic assembly 100 enables a set of one or more energy delivery elements 110 to deliver energy to the treatment site within the blood vessel (604). The set of one or more energy delivery elements 110 may be a single energy delivery element, such as the energy delivery element 110a, 110b, 110c or 110d, or a combination of multiple energy delivery elements, such as a combination of two energy delivery elements 110a-b, or a combination of three energy delivery elements 110a-c. For example, the therapeutic assembly 100 may power on the first energy delivery element 110a and leave or power off the second energy delivery element 110b and/or the other energy delivery elements 110c-d.

Once the therapeutic assembly 100 enables the set of one or more energy delivery elements 110, the therapeutic assembly 100 determines the change in the heart rate (606). As described above, the therapeutic assembly 100 may measure the temperature to determine the heart rate. The therapeutic assembly 100 may compare the determined heart rate to the normal heart rate to determine if there has been a change in the heart rate. This allows the therapeutic assembly 100 to determine the effect, or change, that the enabled energy delivery elements 110 have on the heart rate of the patient. The change may be an increase, a decrease or no change of the heart rate in comparison to the normal heart rate of the patient when no energy delivery elements 110 are enabled to delivery energy. The change may also be quantified in terms of the magnitude or amount of change that occurred.

The therapeutic assembly 100 associates the change in the heart rate, or effect, with the set of one or more energy delivery elements 110 that were enabled (608). The therapeutic assembly 100 may then proceed to cycle power to and/or enable different sets of the one or more energy delivery elements 110 (610). The different sets may be a single energy delivery element 110a, 110b, 110c or 110d or a combination of multiple energy delivery elements 110 that are different than the previously enabled energy delivery element or combination of energy delivery elements. The therapeutic assembly 100 cycles through the different possible sets of the one or more energy delivery elements 110, measures the change in the heart rate for each of the different possible sets and associates the measured change in the heart rate with the corresponding set of the one or more energy delivery elements 110.

Once the different possible sets of one or more energy delivery elements 110 are measured for their corresponding change to the heart rate, the therapeutic assembly may determine the one or more energy delivery elements 110 that cause the change, such as the slowing, in the heart rate, by identifying which combination of the one or more energy deliver elements 110 cause the slowing of the heart rate or otherwise decrease the heart rate when activated. Consequently, the trigger or cause of the bradycardia, such as the stimulation of an autonomic nerve that is distinct from the targeted sympathetic nerves, may be identified.

Since each energy delivery element 110 is at a particular location and/or position along the wall of the blood vessel, the therapeutic assembly 100 may be able to identify and store the potential location of the autonomic nerve, such as the vagus nerve, that is distinct from the targeted sympathetic nerves based on an analysis of the enabling of the different sets of the one or more energy delivery elements 110. For example, the set of the one or more energy delivery elements 110 that causes the greatest change, such as the greatest slowing in the heart rate when enabled, is likely the cause of the change in the heart rate. As such, the corresponding positions of the identified one or more energy delivery elements 110 may approximate where the autonomic nerve, such as the vagus nerve, may be located, which results in the slowing of the heart rate when stimulus or energy is applied. At a minimum, the therapeutic assembly 100 may provide feedback to the physician or other health care professional to allow the physician or other health care professional to view the position on the fluoroscope and note where not to ablate.

In some implementations, the therapeutic assembly 100 may cycle through all combinations of single or multiple energy delivery elements 110. In other implementations, the therapeutic assembly 100 only continues cycling through different sets when disabling the energy delivery element 110 does not stop the decline or decrease in the heart rate.

The therapeutic assembly 100 may power off, turn off, or otherwise disable the energy delivery elements 110 that are identified as the cause of the slowing of the heart rate, such as the slowing of the heart rate (612). The therapeutic assembly 100 may power on, turn on, or otherwise enable the other energy delivery elements 110 that are not identified as the cause of the slowing of the heart rate. This prevents bradycardia and allows the heart rate to return to normal. The location of the disabled energy delivery elements 110 may be noted, displayed or otherwise provided to the clinician, and so when the catheter 108 is moved within the blood vessel, an appropriate set of the one or more energy delivery elements 110 may be enabled/disabled to avoid stimulating the location identified as where the autonomic nerve may be potentially located. In some implementations, the analysis and cycling of the different energy delivery elements 110 may be repeated when the catheter 108 is moved.

The therapeutic assembly 100 may identify and display a potential pathway of the autonomic nerve based on the locations identified as where the vagus nerve may be potentially located during multiple sequential ablations (614). The ablation procedure may be repeated, and the locations may be stored and mapped during the sequential ablations to identify the potential pathway of the vagus nerve. The potential pathway may be displayed on the user interface 118 or other display to allow prediction of a region to avoid within the patient.

FIG. 7 is a flow diagram of a process 700 for performing different actions at different threshold heart rates. One or more computers or one or more data processing apparatuses, for example, the processor 302 of the therapeutic assembly 100 of FIG. 1, appropriately programmed, may implement the process 700.

The therapeutic assembly 100 determines the heart rate, as described above (702). Once the therapeutic assembly 100 determines the heart rate, the therapeutic assembly 100 may determine whether the heart rate is less than a first threshold heart rate (704). The first threshold heart rate may be approximately 7%-12% less than the normal heart rate of the patient. The normal heart rate of the patient may be an average of the heart rate of the patient when no energy is being delivered or applied and may be generally between 60 and 100 beats per minute when the patient is resting.

When the therapeutic assembly 100 determines that the heart rate is less than the first threshold heart rate, the therapeutic assembly 100 may notify the clinician that the heart rate is below normal (706). The notification may indicate the heart rate and that the heart rate is slowing. The notification may indicate that if the clinician takes no action and the heart rate continues to decrease, the therapeutic assembly 100 may identify the cause of the slowing and turn off one, some or all of the energy delivery elements 110 to prevent the slowing of the heart rate. Otherwise, if the heart rate is greater than or equal to the first threshold heart rate, the therapeutic assembly 100 may continue to monitor the heart rate during delivery of the energy.

The therapeutic assembly 100 may determine whether the heart rate is less than a second threshold heart rate (708). The second threshold heart rate may be approximately 17%-22% less than the normal heart rate and may indicate an increase in severity in the slowing of the heart rate in comparison to the first threshold heart rate.

When the therapeutic assembly 100 determines that the heart rate is less than the second threshold heart rate, the therapeutic assembly 100 may determine the cause of the slowing of the heart rate (710). The therapeutic assembly 100 may notify the clinician that the therapeutic assembly 100 intends to determine the cause of the slowing of the heart rate and/or automatically determine the cause of the slowing of the heart rate, as described above and in FIG. 6, for example. Otherwise, if the heart rate is greater than or equal to the second threshold heart rate, the therapeutic assembly 100 may continue to monitor the heart rate and/or continue the execution of any previous actions during delivery of the energy. The therapeutic assembly 100 may stop, turn off or otherwise disable the energy delivery elements 110 that are determined to be the cause of the slowing of the heart rate (712).

The therapeutic assembly 100 may determine whether the heart rate is less than a third threshold heart rate (714). The third threshold heart rate may be approximately 27%-32% less than the normal heart rate and may indicate an increase in severity in the slowing of the heart rate in comparison to the first threshold heart rate and the second threshold heart rate.

When the therapeutic assembly 100 determines that the heart rate is less than the third threshold heart rate, the therapeutic assembly 100 may disable or turn off all of the one or more energy delivery elements 110 (716). In some implementations, the therapeutic assembly 100 may also indicate or notify the clinician of the possibility of bradycardia and suggest treatment, such as the administration of atropine, to increase the heart rate of the patient (718).

Exemplary embodiments of the invention have been disclosed in an illustrative style. Accordingly, the terminology employed throughout should be read in a non-limiting manner. The invention is defined by the appended claims.

## Claims

1. A therapeutic assembly (100) for renal denervation, comprising:
a first sensor (112) configured to continuously detect a first temperature or a first impedance at a first location of a wall of a vessel;
a first energy delivery element (110) configured to deliver energy to the wall of the vessel; and
a processor (302) coupled to the sensor (112) and the first energy delivery element (110) and configured to:
determine a heart rate based on the first temperature or the first impedance at the first location of the wall of the vessel,
determine that the heart rate is less than a threshold heart rate that indicates a slowing of the heart rate, and
control the first energy delivery element (110) to adjust the delivery of the energy to the wall of the vessel,
and the assembly further comprising:
a second energy delivery element (110) configured to deliver a second amount of energy to a second location on the wall of the vessel, wherein the first energy delivery element (110) is configured to deliver a first amount of energy to the first location of the wall of the vessel, wherein the processor (302) is configured to:
power off the first energy delivery element (110) and the second energy delivery element (110);
cycle the first energy delivery element (110) and second energy delivery element (110) to determine a cause of the slowing of the heart rate;
power off one of the first energy delivery element (110) or the second energy delivery element (110) based on the cause of the slowing of the heart rate; and
power on the other one of the first energy delivery element (110) or the second energy delivery element (110) based on the cause of the slowing of the heart rate.

2. The therapeutic assembly (100) of claim 1, wherein to control the first energy delivery element (110) the processor (302) is configured to disable the first energy delivery element (110) when the heart rate is less than the threshold heart rate.

3. The therapeutic assembly (100) of claim 1, wherein the processor (302) is configured to:
provide, on a display, a notification or indication to a clinician that the heart rate is less than the threshold heart rate; and
obtain a confirmation to proceed to determine a cause of the slowing of the heart rate.

4. The therapeutic assembly (100) of claim 1, further comprising:
a second sensor (112) configured to detect a second temperature at a second location of the wall of the vessel, wherein the processor (302) is configured to determine the heart rate further based on the second temperature at the second location of the wall of the vessel.

5. The therapeutic assembly (100) of claim 1, further comprising:
a catheter (108) coupled to the first sensor (112) and the first energy delivery element (110) and configured to be intravascularly inserted into the vessel, wherein the vessel is a renal artery and wherein the first sensor (112) is coupled to the first energy delivery element (110).

6. The therapeutic assembly of claim 5, further comprising:
a radio frequency generator (104) configured to deliver the energy to the first energy delivery element (110), wherein the energy is a radiofrequency (RF) signal, wherein the first energy delivery element (110) is an electrode (110).

7. The therapeutic assembly (100) for renal denervation of claim 1, wherein:
the assembly (100) comprises a plurality of sensors (112) including the first sensor (112),
and wherein the assembly (100) comprises a plurality of energy delivery elements configured as electrodes, including the first energy delivery element (110) and the second energy delivery element (110) and configured to deliver energy to the wall of the vessel; and
wherein the processor (302) is coupled to the plurality of sensors (112) and the plurality of electrodes and is further configured to:
power off one of the plurality of electrodes to prevent bradycardia,
wherein the processor (302) is configured to determine a cause of the slowing of the heart rate when the heart rate is less than the threshold heart rate, and wherein the processor (302) is configured to cycle through the plurality of electrodes to determine a cause of the slowing of the heart rate.

8. The therapeutic assembly (100) of claim 7, wherein the processor (302) independently controls an amount of energy delivered by each of the plurality of electrodes.

9. The therapeutic assembly of claim 7, wherein the processor is configured to turn on, turn off or adjust an amount of energy delivered by each of the plurality of electrodes.

10. The therapeutic assembly (100) of claim 7, wherein the first energy delivery element is configured to deliver a first amount of energy to a first location on the wall of the vessel and the second energy delivery element is configured to deliver a second amount of energy to a second location on the wall of the vessel.

11. The therapeutic assembly (100) of claim 7, wherein the plurality of sensors (112) includes a second sensor (112) that is configured to detect a second temperature at a second location of the wall of the vessel, wherein the processor (302) is configured to determine the heart rate further based on the second temperature at the second location of the wall of the vessel.

12. The therapeutic assembly (100) of claim 7, further comprising:
a catheter (108) coupled to the plurality of sensors (112) and the plurality of electrodes and configured to be intravascularly inserted into the vessel, wherein the vessel is a renal artery and wherein the plurality of sensors (112) are coupled to a corresponding electrode of the plurality of electrodes.

13. The therapeutic assembly of claim 7, further comprising: a radio frequency generator (104) configured to deliver the energy to the plurality of electrodes.

## Patentansprüche

1. Therapeutische Anordnung (100) bei Nierendenervation, umfassend:
einen ersten Sensor (112), der ausgestaltet ist zum kontinuierlichen Erfassen einer ersten Temperatur oder einer ersten Impedanz an einer ersten Stelle einer Gefäßwand;
ein erstes Energieabgabeelement (110), das ausgestaltet ist zum Abgeben von Energie an die Gefäßwand; und
einen Prozessor (302), der mit dem Sensor (112) und dem ersten Energieabgabeelement (110) gekoppelt ist und ausgestaltet ist zum:
Bestimmen einer Herzfrequenz basierend auf der ersten Temperatur oder der ersten Impedanz an der ersten Stelle der Gefäßwand,
Bestimmen, dass die Herzfrequenz unter einem Herzfrequenz-Schwellenwert liegt, der eine Verlangsamung der Herzfrequenz anzeigt, und
Steuern des ersten Energieabgabeelements (110), um die Abgabe der Energie an die Gefäßwand einzustellen,
und wobei die Anordnung ferner umfasst:
ein zweites Energieabgabeelement (110), das ausgestaltet ist zum Abgeben einer zweiten Energiemenge an eine zweite Stelle der Gefäßwand, wobei das erste Energieabgabeelement (110) ausgestaltet ist zum Abgeben einer ersten Energiemenge an die erste Stelle der Gefäßwand, wobei der Prozessor (302) ausgestaltet ist zum:
Abschalten des ersten Energieabgabeelements (110) und des zweiten Energieabgabeelements (110);
Zirkulieren des ersten Energieabgabeelements (110) und des zweiten Energieabgabeelements (110), um eine Ursache für die Verlangsamung der Herzfrequenz zu bestimmen;
Abschalten entweder des ersten Energieabgabeelements (110) oder des zweiten Energieabgabeelements (110) basierend auf der Ursache für die Verlangsamung der Herzfrequenz; und
Einschalten des jeweils anderen von erstem Energieabgabeelement (110) und zweiten Energieabgabeelement (110) basierend auf der Ursache für die Verlangsamung der Herzfrequenz.

2. Therapeutische Anordnung (100) nach Anspruch 1, wobei zum Steuern des ersten Energieabgabeelements (110) der Prozessor (302) ausgestaltet ist, das erste Energieabgabeelement (110) zu deaktivieren, wenn die Herzfrequenz unter dem Herzfrequenz-Schwellenwert liegt.

3. Therapeutische Anordnung (100) nach Anspruch 1, wobei der Prozessor (302) ausgestaltet ist zum:
Bereitstellen, auf einer Anzeige, einer Benachrichtigung oder Anzeige für einen Kliniker, dass die Herzfrequenz unter dem Herzfrequenz-Schwellenwert liegt; und
Erhalten einer Bestätigung, mit dem Bestimmen der Ursache für die Verlangsamung der Herzfrequenz fortzufahren.

4. Therapeutische Anordnung (100) nach Anspruch 1, ferner umfassend:
einen zweiten Sensor (112), der ausgestaltet ist zum Erfassen einer zweiten Temperatur an einer zweiten Stelle der Gefäßwand, wobei der Prozessor (302) ausgestaltet ist, die Herzfrequenz ferner basierend auf der zweiten Temperatur an der zweiten Stelle der Gefäßwand zu bestimmen.

5. Therapeutische Anordnung (100) nach Anspruch 1, ferner umfassend:
einen Katheter (108), der mit dem ersten Sensor (112) und dem ersten Energieabgabeelement (110) gekoppelt und ausgestaltet ist, intravaskulär in das Gefäß eingeführt zu werden, wobei das Gefäß eine Nierenarterie ist und wobei der erste Sensor (112) mit dem ersten Energieabgabeelement (110) gekoppelt ist.

6. Therapeutische Anordnung nach Anspruch 5, ferner umfassend:
einen Hochfrequenz-Generator (104), der ausgestaltet ist zum Abgeben der Energie an das erste Energieabgabeelement (110), wobei die Energie ein Hochfrequenz (HF) -Signal ist, wobei das erste Energieabgabeelement (110) eine Elektrode (110) ist.

7. Therapeutische Anordnung (100) bei Nierendenervation nach Anspruch 1, wobei:
die Anordnung (100) mehrere Sensoren (112) einschließlich des ersten Sensors (112) umfasst,
und wobei die Anordnung (100) mehrere Energieabgabeelemente umfasst, die als Elektroden ausgestaltet sind, einschließlich des ersten Energieabgabeelements (110) und des zweiten Energieabgabeelements (110), und die ausgestaltet sind, Energie an die Gefäßwand abzugeben; und
wobei der Prozessor (302) mit den mehreren Sensoren (112) und den mehreren Elektroden gekoppelt ist und ferner ausgestaltet ist zum:
Abschalten einer der mehreren Elektroden, um eine Bradykardie zu verhindern,
wobei der Prozessor (302) ausgestaltet ist, eine Ursache für die Verlangsamung der Herzfrequenz zu bestimmen, wenn die Herzfrequenz unter dem Herzfrequenz-Schwellenwert liegt, und wobei der Prozessor (302) ausgestaltet ist, durch die mehreren Elektroden zu zirkulieren, um eine Ursache für die Verlangsamung der Herzfrequenz zu bestimmen.

8. Therapeutische Anordnung (100) nach Anspruch 7, wobei der Prozessor (302) unabhängig eine Energiemenge regelt, die von jeder der mehreren Elektroden abgegeben wird.

9. Therapeutische Anordnung nach Anspruch 7, wobei der Prozessor ausgestaltet ist, die mehreren Elektroden einzuschalten, abzuschalten oder eine von diesen abgegebene Energiemenge einzustellen.

10. Therapeutische Anordnung (100) nach Anspruch 7, wobei das erste Energieabgabeelement ausgestaltet ist zum Abgeben einer ersten Energiemenge an eine erste Stelle der Gefäßwand und das zweite Energieabgabeelement ausgestaltet ist zum Abgeben einer zweiten Energiemenge an eine zweite Stelle der Gefäßwand.

11. Therapeutische Anordnung (100) nach Anspruch 7, wobei die mehreren Sensoren (112) einen zweiten Sensor (112) einschließen, der ausgestaltet ist zum Erfassen einer zweiten Temperatur an einer zweiten Stelle der Gefäßwand, wobei der Prozessor (302) ausgestaltet ist, die Herzfrequenz ferner basierend auf der zweiten Temperatur an der zweiten Stelle der Gefäßwand zu bestimmen.

12. Therapeutische Anordnung (100) nach Anspruch 7, ferner umfassend:
einen Katheter (108), der mit den mehreren Sensoren (112) und den mehreren Elektroden gekoppelt ist und ausgestaltet ist, intravaskulär in das Gefäß eingeführt zu werden, wobei das Gefäß eine Nierenarterie ist und wobei die mehreren Sensoren (112) mit einer entsprechenden Elektrode der mehreren Elektroden gekoppelt sind.

13. Therapeutische Anordnung nach Anspruch 7, ferner umfassend: einen Hochfrequenz-Generator (104), der ausgestaltet ist zum Abgeben der Energie an die mehreren Elektroden.

## Revendications

1. Ensemble thérapeutique (100) pour la dénervation rénale, comprenant :
un premier capteur (112) configuré pour détecter en continu une première température ou une première impédance à un premier emplacement d'une paroi d'un vaisseau ;
un premier élément d'apport d'énergie (110) configuré pour apporter de l'énergie à la paroi du vaisseau ; et
un processeur (302) couplé au capteur (112) et au premier élément d'apport d'énergie (110) et configuré pour :
déterminer une fréquence cardiaque sur la base de la première température ou de la première impédance au premier emplacement de la paroi du vaisseau,
déterminer que la fréquence cardiaque est inférieure à un seuil de fréquence cardiaque qui indique un ralentissement de la fréquence cardiaque, et
commander le premier élément d'apport d'énergie (110) pour ajuster l'apport d'énergie à la paroi du vaisseau, et l'ensemble comprenant en outre :
un second élément d'apport d'énergie (110) configuré pour apporter une seconde quantité d'énergie à un second emplacement sur la paroi du vaisseau, le premier élément d'apport d'énergie (110) étant configuré pour apporter une première quantité d'énergie au premier emplacement de la paroi du vaisseau, le processeur (302) étant configuré pour :
mettre hors tension le premier élément d'apport d'énergie (110) et le second élément d'apport d'énergie (110) ;
faire fonctionner en cycle le premier élément d'apport d'énergie (110) et le second élément d'apport d'énergie (110) afin de déterminer une cause du ralentissement de la fréquence cardiaque ;
mettre hors tension l'un du premier élément d'apport d'énergie (110) ou du second élément d'apport d'énergie (110) sur la base de la cause du ralentissement de la fréquence cardiaque ; et
mettre sous tension l'autre du premier élément d'apport d'énergie (110) ou du second élément d'apport d'énergie (110) sur la base de la cause du ralentissement de la fréquence cardiaque.

2. Ensemble thérapeutique (100) selon la revendication 1, pour commander le premier élément d'apport d'énergie (110) le processeur (302) étant configuré pour désactiver le premier élément d'apport d'énergie (110) lorsque la fréquence cardiaque est inférieure au seuil de fréquence cardiaque.

3. Ensemble thérapeutique (100) selon la revendication 1, le processeur (302) étant configuré pour :
fournir, sur un dispositif d'affichage, une notification ou indication à un clinicien que la fréquence cardiaque est inférieure au seuil de fréquence cardiaque ; et
obtenir une confirmation pour procéder à la détermination de cause du ralentissement de la fréquence cardiaque.

4. Ensemble thérapeutique (100) selon la revendication 1, comprenant en outre :
un second capteur (112) configuré pour détecter une seconde température à un second emplacement de la paroi du vaisseau, le processeur (302) étant configuré pour déterminer la fréquence cardiaque en outre sur la base de la seconde température au second emplacement de la paroi du vaisseau.

5. Ensemble thérapeutique (100) selon la revendication 1, comprenant en outre :
un cathéter (108) couplé au premier capteur (112) et au premier élément d'apport d'énergie (110) et configuré pour être inséré de manière intravasculaire dans le vaisseau, le vaisseau étant une artère rénale et le premier capteur (112) étant couplé au premier élément d'apport d'énergie (110).

6. Ensemble thérapeutique selon la revendication 5, comprenant en outre :
un générateur de radiofréquence (104) configuré pour apporter l'énergie au premier élément d'apport d'énergie (110), l'énergie étant un signal de radiofréquence (RF), le premier élément d'apport d'énergie (110) étant une électrode (110).

7. Ensemble thérapeutique (100) pour la dénervation rénale selon la revendication 1 :
l'ensemble (100) comprenant une pluralité de capteurs (112) incluant le premier capteur (112),
et l'ensemble (100) comprenant une pluralité d'éléments d'apport d'énergie configurés comme électrodes, incluant le premier élément d'apport d'énergie (110) et le second élément d'apport d'énergie (110) et configurés pour apporter de l'énergie à la paroi du vaisseau ; et
le processeur (302) étant couplé à la pluralité de capteurs (112) et à la pluralité d'électrodes et étant en outre configuré pour :
mettre hors tension l'une de la pluralité d'électrodes pour prévenir la bradycardie,
le processeur (302) étant configuré pour déterminer une cause du ralentissement de la fréquence cardiaque lorsque la fréquence cardiaque est inférieure au seuil de fréquence cardiaque, et le processeur (302) étant configuré pour faire fonctionner en cycle la pluralité d'électrodes pour déterminer une cause du ralentissement de la fréquence cardiaque.

8. Ensemble thérapeutique (100) selon la revendication 7, le processeur (302) commandant de manière indépendante une quantité d'énergie apportée par chacune de la pluralité d'électrodes.

9. Ensemble thérapeutique selon la revendication 7, le processeur étant configuré pour allumer, éteindre ou ajuster une quantité d'énergie apportée par chacune de la pluralité d'électrodes.

10. Ensemble thérapeutique (100) selon la revendication 7, le premier élément d'apport d'énergie étant configuré pour apporter une première quantité d'énergie à un premier emplacement sur la paroi du vaisseau et le second élément d'apport d'énergie étant configuré pour apporter une seconde quantité d'énergie à un second emplacement sur la paroi du vaisseau.

11. Ensemble thérapeutique (100) selon la revendication 7, la pluralité de capteurs (112) incluant un second capteur (112) qui est configuré pour détecter une seconde température à un second emplacement de la paroi du vaisseau, le processeur (302) étant configuré pour déterminer la fréquence cardiaque en outre sur la base de la seconde température au second emplacement de la paroi du vaisseau.

12. Ensemble thérapeutique (100) selon la revendication 7, comprenant en outre :
un cathéter (108) couplé à la pluralité de capteurs (112) et à la pluralité d'électrodes et configuré pour être inséré de manière intravasculaire dans le vaisseau, le vaisseau étant une artère rénale et la pluralité de capteurs (112) étant couplée à une électrode correspondante de la pluralité d'électrodes.

13. Ensemble thérapeutique selon la revendication 7, comprenant en outre : un générateur de radiofréquence (104) configuré pour apporter l'énergie à la pluralité d'électrodes.
